# EUROPEAN PATENT APPLICATION

(11) **EP 4 144 409 A1**
(43) Date of publication of application: **08.03.2023**
(21) Application number: 22193172.8
(22) Date of filing: 31.08.2022
(51) Int. Cl.: A61N 1/378, A61N 1/36, A61N 1/05

(54) **ELECTRICAL STIMULATION DEVICE AND ELECTRICAL STIMULATION SYSTEM**

(30) Priority: 03.09.2021 CN 202111031089
(71) Applicant: Gimer Medical. Co., Ltd, New Taipei City 221 (TW)
(72) Inventor: PAN, Jian-Hao, 221 New Taipei City (TW)
(74) Representative: Ter Meer Steinmeister & Partner

(57) **Abstract**

An electrical stimulation device includes a signal receiving circuit, a rectifying circuit and a signal processing circuit. The signal receiving circuit receives and outputs a frequency signal. The rectifying circuit receives the frequency signal and rectifies the frequency signal to generate a rectifying signal. The signal processing circuit receives the rectifying signal to generate an electrical stimulation signal.

## Description

This Application claims priority of China Patent Application No. 202111031089.4, filed on September 3, 2021.

### BACKGROUND

### Technology Field

The present disclosure relates to an electrical stimulation device, and, in particular, to an electrical stimulation device and an electrical stimulation system.

### Description of the Related Art

In recent years, dozens of therapeutic nerve electrical stimulation devices have been developed, and at least tens of thousands of people undergo electrical stimulation device implantation every year. Due to the development of precision manufacturing technology, the size of medical devices has been miniaturized and may be implanted inside the human body, for example, an implantable electrical stimulation device.

However, some of the current implantable electrical stimulation devices use an implantable electrical stimulator without batteries, instead using an external controller to provide electrical energy to the implantable electrical stimulator so that it can provide electrical stimulation in the manner of a wireless power supply. The external controller and the implantable electrical stimulator without batteries must be precisely aligned, otherwise the implantable electrical stimulator may not receive electrical energy. In addition, when the implantable electrical stimulator is implanted deep, the efficiency of the implantable electrical stimulator to receive electrical energy may be greatly decreased. Furthermore, the implantable electrical stimulator can easily be induced by mistake. As long as the implantable electrical stimulator is close to an unspecified emission source with a similar electromagnetic wave, the implantable electrical stimulator may be immediately induced to perform electrical stimulation, thereby causing trouble for the user. Therefore, how to effectively improve the design of the implantable electrical stimulation device to decrease the complexity of circuit design, decrease the size of the electrical stimulation device and increase the convenience of use has become an important issue.

### SUMMARY

It is an object of the present disclosure to provide an electrical stimulation device and an electrical stimulation system having a decreased complexity of the circuit design, being decreased in size of the electrical stimulation device, and providing an increased convenience of use.

The object is solved by the features of the independent claims. Preferred embodiments are given in the dependent claims.

An embodiment of the present disclosure provides an electrical-stimulation device, which includes a signal receiving circuit, a rectifying circuit and a signal processing circuit. The signal receiving circuit is configured to receive and output a frequency signal. The rectifying circuit is configured to receive the frequency signal and rectify the frequency signal to generate a rectifying signal. The signal processing circuit is configured to receive the rectifying signal to generate an electrical stimulation signal.

In one or more embodiments, the electrical stimulation device may further comprise at least one electrode.

In one or more embodiments, the at least one electrode may be coupled to the signal processing circuit.

In one or more embodiments, the signal processing circuit may be an oscillator.

In one or more embodiments, the electrical stimulation device may not have a battery.

In one or more embodiments, the signal processing circuit may comprise a counter or a pulse width modulation circuit.

In one or more embodiments, the electrical stimulation device may further comprise a signal converting circuit, configured to receive the electrical stimulation signal, and to convert the electrical stimulation signal to generate a sine wave electrical stimulation signal.

In one or more embodiments, the signal converting circuit may be a passive filter.

In one or more embodiments, the signal converting circuit may comprise a first inductor, a first capacitor, a second inductor, a second capacitor, a third capacitor, a third inductor.

In one or more embodiments, the first inductor may comprise a first terminal and a second terminal.

In one or more embodiments, the first terminal of the first inductor may be coupled to the signal processing circuit and receives the electrical stimulation signal.

In one or more embodiments, the first capacitor may comprise a first terminal and a second terminal, wherein the first terminal of the first capacitor may be coupled to the second terminal of the first inductor.

In one or more embodiments, the second inductor may comprising a first terminal and a second terminal, wherein the first terminal of the second inductor may be coupled to the second terminal of the first capacitor, and the second terminal of the second inductor may be coupled to a ground terminal.

In one or more embodiments, the second capacitor may comprise a first terminal and a second terminal, wherein the first terminal of the second capacitor may be coupled to the first terminal of the second inductor, and the second terminal of the second capacitor may be coupled to the second terminal of the second inductor.

In one or more embodiments, the third capacitor may comprising a first terminal and a second terminal, wherein the first terminal of the third capacitor may be is coupled to the second terminal of the first capacitor.

In one or more embodiments, the third inductor may comprise a first terminal and a second terminal, wherein the first terminal of the third inductor may be coupled to the second terminal of the third capacitor, and the second terminal of the third inductor may generate the sine wave electrical stimulation signal.

In one or more embodiments, the signal converting circuit may be an active filter.

In one or more embodiments, the signal converting circuit may comprise a first resistor, a second resistor, a first capacitor, a second capacitor, a third resistor, and an amplifier.

In one or more embodiments, the first resistor may comprising a first terminal and a second terminal, wherein the first terminal of the first resistor may be coupled to the signal processing circuit and may receive the electrical stimulation signal.

In one or more embodiments, the second resistor may comprise a first terminal and a second terminal, wherein the first terminal of the second resistor may be coupled to the second terminal of the first resistor, and the second terminal of the second resistor may be coupled to a ground.

In one or more embodiments, the first capacitor may comprise a first terminal and a second terminal, wherein the first terminal of the first capacitor may be coupled to the second terminal of the first resistor.

In one or more embodiments, the second capacitor may comprise a first terminal and a second terminal, wherein the first terminal of the second capacitor may be coupled to the first terminal of the first capacitor.

In one or more embodiments, the third resistor may comprise a first terminal and a second terminal, wherein the first terminal of the third resistor may be coupled to the second terminal of the first resistor, and the second terminal of the third resistor may be coupled to the second terminal of the second capacitor.

In one or more embodiments, the amplifier may comprise a first input terminal, a second input terminal and an output terminal.

In one or more embodiments, the first input terminal of the amplifier may be coupled to the ground terminal, the second input terminal of the amplifier may be coupled to the second terminal of the third resistor, and the output terminal of the amplifier may be coupled to the first terminal of the third resistor and outputs the sine wave electrical stimulation signal.

In one or more embodiments, the electrical stimulation device may further comprise a protection circuit, coupled to the signal processing circuit, wherein the protection circuit may be configured to determine whether to output the electrical stimulation signal.

In one or more embodiments, the protection circuit may be a Hall switch or a magnetic reed switch.

In one or more embodiments, the protection circuit may comprise a processing circuit and a switch.

In one or more embodiments, the processing circuit may be coupled to the signal receiving circuit and may be configured to receive a triggering signal to generate a processing signal.

In one or more embodiments, the switch may be coupled to the signal processing circuit and the processing circuit, and may be configured to receive the processing signal and determine whether to output the electrical simulation signal according to the processing signal.

In one or more embodiments, the processing circuit may be is a near field communication circuit, a radio frequency identification circuit, or an authenticator and security integrated circuit.

In one or more embodiments, the electrical stimulation device may further comprise at least one electrode, wherein the at least one electrode may be coupled to the protection circuit.

In one or more embodiments, the signal receiving circuit may comprise a coil, an antenna, an ultrasonic circuit, or a combination thereof.

In one or more embodiments, a frequency range of the frequency signal may comprise a range of 1 MHz to 1.2 MHz, a range of 9 KHz to 200 KHz, or a range of 6 MHz to 45 MHz.

In one or more embodiments, a frequency of the frequency signal may be 13.56 MHz.

In one or more embodiments, a frequency range of the electrical simulation signal may be 100 KHz to 2000 KHz.

In one or more embodiments, a frequency range of the electrical simulation signal may be 480 KHz to 520 KHz.

In one or more embodiments, a voltage range of the electrical stimulation signal may be between -25 V and 25 V.

In one or more embodiments, the current range of the electrical simulation signal may be between 0 and 60 mA.

In addition, an embodiment of the present disclosure provides an electrical-stimulation system, which includes an external controller and an electrical stimulation device. The external controller includes a signal generating circuit and a signal transmitting circuit. The signal generating circuit is configured to generate a frequency signal. The signal transmitting circuit is configured to receive and transmit the frequency signal. The electrical stimulation device includes a signal receiving circuit, a rectifying circuit and a signal processing circuit. The signal receiving circuit is configured to receive and output the frequency signal. The rectifying circuit is configured to receive the frequency signal and rectify the frequency signal to generate a rectifying signal. The signal processing circuit is configured to receive the rectifying signal to generate an electrical stimulation signal.

In one or more embodiments, the electrical stimulation system may further comprise a signal converting circuit, configured to receive the electrical stimulation signal, and to convert the electrical stimulation signal to generate a sine wave electrical stimulation signal.

In one or more embodiments, the electrical stimulation system may further comprise a protection circuit, coupled to the signal processing circuit, wherein the protection circuit is configured to determine whether to output the electrical stimulation signal.

In one or more embodiments, the protection circuit may be a Hall switch or a magnetic reed switch.

In one or more embodiments, the protection circuit may comprise a processing circuit, coupled to the signal receiving circuit, and may be configured to receive a triggering signal to generate a processing signal; and a switch, coupled to the signal processing circuit and the processing circuit, and may be configured to receive the processing signal and determine whether to output the electrical simulation signal according to the processing signal.

In one or more embodiments, the processing circuit may be a near field communication circuit, a radio frequency identification circuit, or an authenticator and security integrated circuit.

In one or more embodiments, the signal transmitting circuit and the signal receiving circuit respectively may comprise a coil, an antenna, an ultrasonic circuit, or a combination thereof.

In one or more embodiments, a frequency range of the frequency signal may comprise a range of 1 MHz to 1.2 MHz, a range of 9 KHz to 200 KHz, or a range of 6 MHz to 45 MHz.

In one or more embodiments, a frequency range of the electrical simulation signal may be 100 KHz to 2000 KHz.

According to the electrical stimulation device and the electrical stimulation system disclosed by the present disclosure, the signal receiving circuit receives and outputs the frequency signal, the rectifying circuit receives the frequency signal and rectifies the frequency signal to generate the rectifying signal, and the signal processing circuit receives the rectifying signal to generate the electrical stimulation signal. Therefore, the complexity of circuit design may be effectively decreased, the size of the electrical stimulation device is decreased, and the convenience of use is increased.

### BRIEF DESCRIPTION OF THE DRAWINGS

The present disclosure can be more fully understood by reading the subsequent detailed description and examples with references made to the accompanying drawings, wherein:
FIG. 1 is a schematic view of an electrical stimulation system according to an embodiment of the present disclosure;
FIG. 2 is a schematic view of an electrical stimulation device according to an embodiment of the present disclosure;
FIG. 3 is a schematic view of an electrical stimulation device according to another embodiment of the present disclosure;
FIG. 4 is a waveform diagram of an electrical stimulation signal of an electrical stimulation device according to an embodiment of the present disclosure;
FIG. 5 is a schematic view of an electrical stimulation system according to another embodiment of the present disclosure;
FIG. 6 is a schematic view of an electrical stimulation system according to another embodiment of the present disclosure; and
FIG. 7 is a schematic view of an electrical stimulation system according to another embodiment of the present disclosure.

### DETAILED DESCRIPTION OF THE DISCLOSURE

Technical terms of the present disclosure are based on general definition in the technical field of the present disclosure. If the present disclosure describes or explains one or some terms, definition of the terms is based on the description or explanation of the present disclosure. Each of the disclosed embodiments has one or more technical features. In possible implementation, a person skilled in the art would selectively implement all or some technical features of any embodiment of the present disclosure or selectively combine all or some technical features of the embodiments of the present disclosure.

In each of the following embodiments, the same reference number represents the same or a similar element or component.

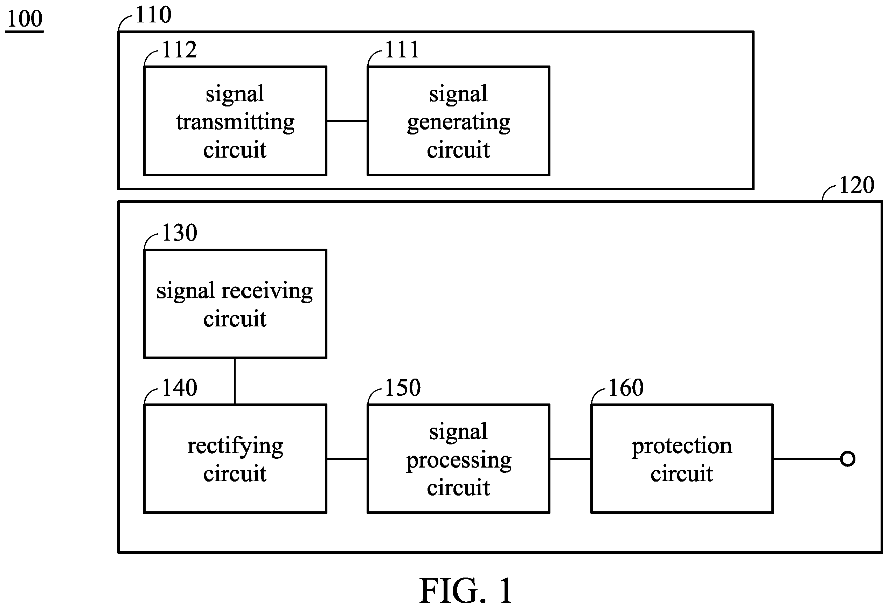

FIG. 1 is a schematic view of an electrical stimulation system according to an embodiment of the present disclosure. Please refer to FIG. 1. The electrical stimulation system 100 includes an external controller 110 and an electrical stimulation device 120. The external controller 110 at least includes a signal generating circuit 111 and a signal transmitting circuit 112. The signal generating circuit 111 is configured to generate a frequency signal. The signal transmitting circuit 112 is coupled to the signal generating circuit 111, and configured to transmit or transmit/receive the frequency signal. In some embodiments, the signal transmitting circuit 112 may include a coil, an antenna, an ultrasonic circuit, or a combination thereof, but the embodiment of the present disclosure is not limited thereto.

In addition, in some embodiments, the frequency range of the frequency signal may be between 1 MHz to 1.2 MHz, like a general energy transmission frequency, but the embodiment of the present disclosure is not limited thereto. In some embodiments, the frequency range of the frequency signal may be between 9 KHz to 200 KHz, like an extremely low energy transmission frequency, but the embodiment of the present disclosure is not limited thereto. In some embodiments, the frequency range of the frequency signal may be between 6 MHz to 45 MHz, like an industrial scientific medical band (ISM Band) regulated by the federal communications commission (FCC), but the embodiment of the present disclosure is not limited thereto. Furthermore, the frequency of the frequency signal may be 6.78 MHz, 13.56 MHz, 27.12 MHz, or 40.68 MHz, but the embodiment of the present disclosure is not limited thereto.

In the embodiment, the electrical stimulation device 120 is an implanted device and does not have a battery. That is, the electrical stimulation device 120 may be implanted inside the human body, and the external controller 110 may be configured outside the human body. Therefore, the electrical stimulation device 120 of the embodiment does not require any control unit, thereby decreasing the numbers of the circuit components in use and decreasing the complexity of circuit design. In addition, the external controller 110 may be configured at a position where the electrical stimulation device 120 may receive the frequency signal, and the external controller 110 does not need to be precisely aligned with the electrical stimulation device 120, thereby increasing the convenience of use.

The electrical stimulation device 120 includes a signal receiving circuit 130, a rectifying circuit 140 and a signal processing circuit 150. The signal receiving circuit 130 is configured to receive and output the frequency signal. In the embodiment, the signal receiving circuit 130 may include a coil, an antenna, an ultrasonic circuit, or a combination thereof, but the embodiment of the present disclosure is not limited thereto. For example, the signal receiving circuit 130 receives the frequency signal transmitted by the signal transmitting circuit 112 in a wireless manner, and output the received frequency signal. In some embodiments, when the signal transmitting circuit 112 and the signal receiving circuit 130 respectively include the coil, the energy transmission manner of the signal transmitting circuit 112 and the signal receiving circuit 130 may be an electromagnetic induction or an electromagnetic resonance. In some embodiments, when the signal transmitting circuit 112 and the signal receiving circuit 130 respectively include the antenna, the energy transmission manner of the signal transmitting circuit 112 and the signal receiving circuit 130 may be in the form of radio wave. In some embodiments, when the signal transmitting circuit 112 includes an ultrasonic generator and the signal receiving circuit 130 includes an ultrasonic transducer, the energy transmission manner of the signal transmitting circuit 112 and the signal receiving circuit 130 may be in the form of ultrasonic energy transmission.

The rectifying circuit 140 is coupled to the signal receiving circuit 130. The rectifying circuit 140 is configured to receive the frequency signal and rectify the frequency signal to generate a rectifying signal. In the embodiment, the rectifying circuit 140 may be a bridge rectifier, such as a half-bridge rectifier or a full-bridge rectifier, but the embodiment of the present disclosure is not limited thereto.

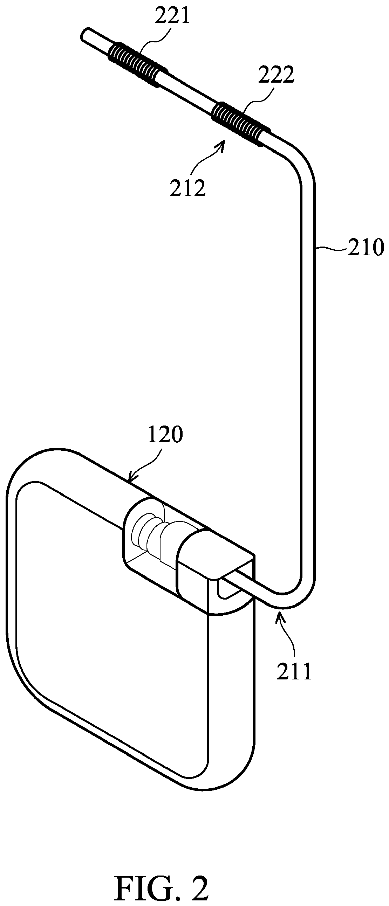

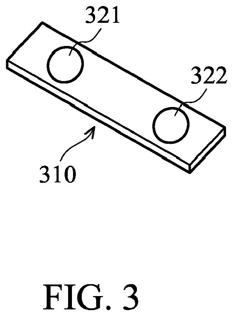

The signal processing circuit 150 is coupled to the rectifying circuit 140. The signal processing circuit 150 is configured to receive the rectifying signal to generate an electrical stimulation signal. In some embodiments, the signal processing circuit 150 may include a timer, which is used for frequency multiplication or frequency division, but the embodiment of the present disclosure is not limited thereto. In some embodiments, the signal processing circuit 150 may be an oscillator, but the embodiment of the present disclosure is not limited thereto. In addition, the above electrical stimulation signal is, for example, a sine wave signal. That is, in some embodiments, the rectifying signal is adjusted through the signal processing circuit 150 to generate the electrical stimulation signal with the sine wave signal output. Then, the electrical stimulation signal may be output to the lead 210, such that the lead 210 may transmit the electrical stimulation signal a target area to be stimulated corresponding to an electrode 221 or an electrode 222 of the lead 210, so as to perform an electrical stimulation operation in the target area, as shown in FIG. 2. As shown in FIG. 3, in some embodiments, the signal receiving circuit 130, the rectifying circuit 140, the signal processing circuit 150 and the protection circuit 160 in FIG. 1 may be configured in an electrical stimulation device 310 in a packaged manner, and an electrode 321 and an electrode 322 may be configured on one side of the electrical stimulation device 310. The rectifying signal is adjusted through the signal processing circuit 150 to generate the electrical stimulation signal with the sine wave signal output. The electrical stimulation signal may be transmitted to a target area to be stimulated corresponding to the electrode 321 or the electrode 322, so as to perform an electrical stimulation operation in the target area. In some embodiments, the electrical stimulation device 310 is a flat rectangular parallelepiped with a length of 3 cm, a width of 1 cm, and a height of 0.5 cm. In addition, the electrical stimulation device 310 of FIG. 3 may be applied to nerves in the shallower layer of human tissue, such as tibial nerve.

In some embodiments, the signal processing circuit 150 is, for example, a counter or a pulse width modulation (PWM) circuit, but the embodiment of the present disclosure is not limited thereto. In addition, the above electrical stimulation signal is, for example, a square wave signal. That is, in some embodiments, the rectifying signal is adjusted through the signal processing circuit 150 to generate the electrical stimulation signal with the square wave signal output. Then, the electrical stimulation signal may be output to the lead 210, such that the lead 210 may transmit the electrical stimulation signal the target area to be stimulated corresponding to the electrode 221 or the electrode 222 of the lead 210, , so as to perform the electrical stimulation operation in the target area, as shown in FIG. 2. In some embodiments, the rectifying signal is adjusted through the signal processing circuit 150 to generate the electrical stimulation signal with the square wave signal output, and the electrical stimulation signal is transmitted to the target area to be stimulated corresponding to the electrode 321 or the electrode 322, so as to perform the electrical stimulation operation in the target area, as shown in FIG. 3. In addition, in the above embodiment, the electrical stimulation signal is the square wave signal as an example, but the embodiment of the present disclosure is not limited thereto. In some embodiments, the electrical stimulation signal may also be a triangular wave, a pulse wave, etc.

In some embodiments, the frequency range of the above electrical stimulation signal is, for example, 100 KHz to 2000 KHz. In some embodiments, the frequency range of the electrical stimulation signal is, for example, 200 KHz to 800 KHz. In some embodiments, the frequency range of the electrical stimulation signal is, for example, 480 KHz to 520 KHz. In some embodiments, the frequency of the electrical stimulation signal is, for example, 500 KHz. In addition, the frequency of the electrical stimulation signal and the frequency of the frequency signal may be the same or different.

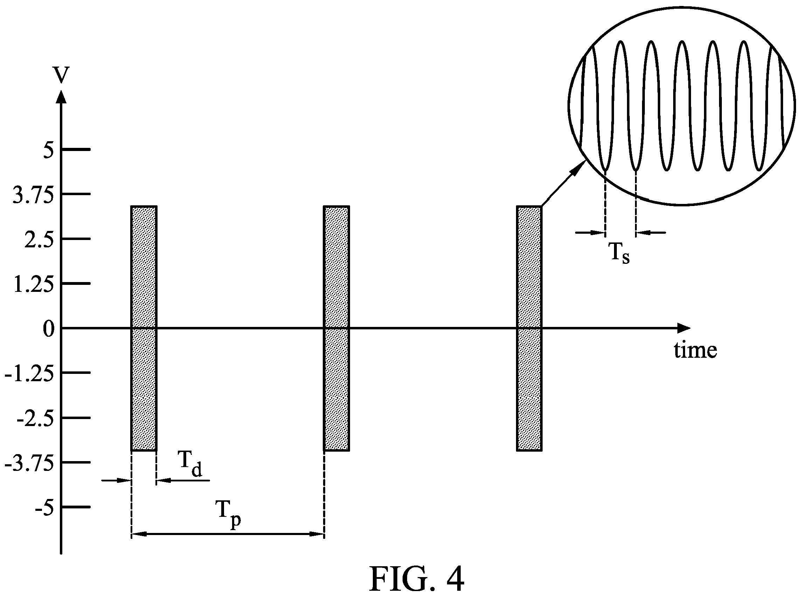

In some embodiments, as shown in FIG. 4, the above electrical stimulation signal may be a pulsed radio-frequency (PRF) signal (or referred to as a pulse signal), a continuous sine wave, a continuous triangular wave, etc., but the embodiment of the present disclosure is not limited thereto. In addition, when the electrical stimulation signal is a pulse alternating signal, one pulse cycle time Tₚ includes a plurality of pulse signals and at least one rest period of time, and the pulse cycle time Tₚ is the reciprocal of the pulse repetition frequency. The pulse repetition frequency range (also referred to as the pulse frequency range) is, for example, between 0 and 1 KHz, preferably between 1 and 100 Hz. In the embodiment, the pulse repetition frequency of the electrical stimulation signal is, for example, 2 Hz. In addition, the duration time T_{d} of the plurality of pulses in one pulse cycle time is, for example, between 1 and 250 milliseconds (ms), preferably between 10 and 100ms. In the embodiment, the duration time T_{d} is, for example, 25 ms. In the embodiment, the frequency of the electrical stimulation signal is 500 KHz, in other words, the cycle time Tₛ of the electrical stimulation signal is about 2 microseconds (µs). Furthermore, the frequency of the above electrical stimulation signal is the intra-pulse frequency in each pulse alternating signal of FIG. 4. Moreover, the voltage range of the above electrical stimulation signal may be between -25 V and 25 V. Furthermore, the voltage range of the above electrical stimulation signal may further be between -20V and 20V. The current range of the above electrical stimulation signal may be between 0 and 60 mA. Furthermore, the current range of the above electrical stimulation signal may further be between 0 and 50 mA.

In the embodiment, the electrical stimulation device 120 further includes a protection circuit 160. The protection circuit 160 is coupled to the signal processing circuit 150, and the protection circuit 160 is configured to determine whether to output the electrical stimulation signal generated by the signal processing circuit 150. In some embodiments, the protection circuit 160 is, for example, a Hall switch or a magnetic reed switch, but the embodiment of the present disclosure is not limited thereto. For example, when the external controller 110 is configured with a triggering component (such as a magnet), the protection circuit 160 (such as the Hall switch or the magnetic reed switch) may sense the magnetic field generated by the triggering component (such as the magnet) and be turned on, so as to output the electrical stimulation signal. In addition, when the external controller 110 is not configured with a triggering component (such as a magnet), the protection circuit 160 (such as the Hall switch or the magnetic reed switch) may not sense the triggering component and not be turned on, and the protection circuit 160 does not output the electrical stimulation signal. Therefore, the situation that the electrical stimulation device 120 senses or receives the frequency signal generated by the other devices or emission sources to generate the error operation (for example, directly perform the electrical stimulation operation) may be effectively avoided, so as to increase the safety of the electrical stimulation device 120 and decrease the trouble of the user.

Furthermore, in the embodiment, the external controller 110 may be configured outside the human body, and the electrical stimulation device 120 may be implanted inside the human body. When the electrical stimulation device 120 is implanted inside the human body, the electrical stimulation device 120 may be placed under the skin of the human body, and one terminal 211 of the lead 210 is connected to the electrical stimulation device 120, and the other terminal 212 of the lead 210 is placed close to a target area to be stimulated. The electrical stimulation system 100 as a spinal cord electrical stimulation system is taken as an example, the other terminal 212 of at least part of the lead 210 is disposed in the epidural space to electrically stimulate the spinal cord, the spinal nerve or the dorsal root ganglia (DRG). The signal processing circuit 150 of the electrical stimulation device 120 transmits the electrical stimulation signal to an output electrode. That is, the signal processing circuit 150 may transmit the electrical stimulation signal to the output electrode (such as the electrode 221 or the electrode 222) of the other terminal 212 of the lead 210 through the terminal 211 of the lead 210, so as to electrically stimulate the target area. The current transmitted by the electrical stimulation device 120 may flow out from one output electrode (such as the electrode 221 or the electrode 222) of the lead 210, and then conducts through the human tissue, and then flow back to the lead 210 from the other electrode (such as the electrode 222 or the electrode 221). In the embodiment, the electrode 221 and the electrode 222 may be a pair of electrodes. In some embodiments, the electrode 221 may be a positive electrode, and the electrode 222 may be a negative electrode. In some embodiments, the electrode 221 may be the negative electrode, and the electrode 222 may be the positive electrode.

In addition, the target nerve area of electrical stimulation may also be in the brain for electrical stimulation of brain cortex or deep brain stimulation (DBS) or abdominal and peripheral nerves. In some embodiments, the electrical stimulation system 100 may also be used to relieve or treat diseases, such as pain, overactive bladder (OAB), renal hypertension, spasm, premature ejaculation or carpal tunnel syndrome (CTS), etc. In some embodiments, the target nerve area of electrical stimulation may also be a lateral recess or a peripheral nervous system (PNS). Furthermore, the target area of the electrical stimulation may also receive less energy per unit time to ensure the subthreshold stimulation, so that the patient implanted with the electrical-stimulation system may reduce the chance of feeling paresthesia to perform the paresthesia-free treatment.

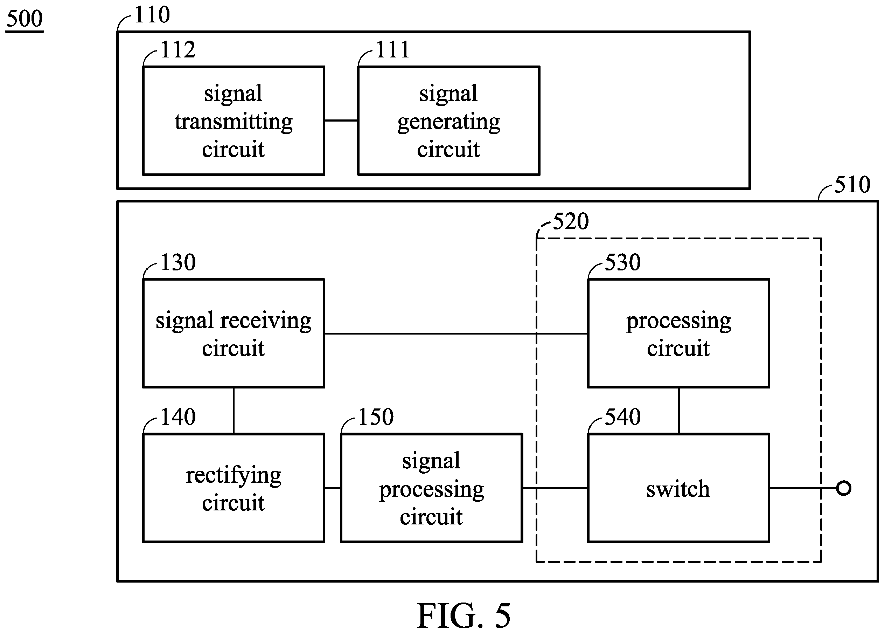

FIG. 5 is a schematic view of an electrical stimulation system according to another embodiment of the present disclosure. Please refer to FIG. 5. The electrical stimulation system 500 includes an external controller 110 and an electrical stimulation device 510. In the embodiment, the external controller 110 in FIG. 5 is the same as or similar to external controller 110 in FIG. 1. Accordingly, the external controller 110 in FIG. 5 may refer to the description of the embodiment of FIG. 1, and the description thereof is not repeated herein.

The electrical stimulation device 510 includes a signal receiving circuit 130, a rectifying circuit 140, a signal processing circuit 150 and a protection circuit 520. In the embodiment, the signal receiving circuit 130, the rectifying circuit 140 and the signal processing circuit 150 in FIG. 5 are the same as or similar to the signal receiving circuit 130, the rectifying circuit 140 and the signal processing circuit 150 in FIG. 1. Accordingly, the signal receiving circuit 130, the rectifying circuit 140 and the signal processing circuit 150 in FIG. 5 may refer to the embodiment of FIG. 1, and the description thereof is not repeated herein.

In the embodiment, the protection circuit 520 is coupled to the signal processing circuit 150, and the protection circuit 520 is configured to determine whether to output the electrical stimulation signal generated by the signal processing circuit 150. Furthermore, the protection circuit 520 includes a processing circuit 530 and a switch 540. The processing circuit 530 is coupled to the signal receiving circuit 130. The processing circuit 530 is configured to receive a triggering signal to generate a processing signal. In the embodiment, the processing circuit 530 is, for example, a near field communication (NFC) circuit, a radio frequency identification (RFID) circuit, or an authenticator and security integrated circuit (IC).

The switch 540 is coupled to the signal processing circuit 150 and the processing circuit 530. The switch 540 is configured to receive the processing signal and determine whether to output the electrical simulation signal according to the processing signal. For example, the switch 540 includes a control terminal, a first terminal and a second terminal. The control terminal of the switch 540 is coupled to the processing circuit 530, such that the switch 540 is controlled by the processing signal generated by the processing circuit 530. The first terminal of the switch 540 is coupled to the signal processing circuit 150. The second terminal of the switch 540 is configured to output the electrical stimulation signal. In some embodiments, the switch 540 is, for example, a metal oxide semiconductor field effect transistor (MOSFET), a bipolar junction transistor (BJT), etc., but the embodiment of the present disclosure is not limited thereto.

In the operation of the protection circuit 520, when the external controller 110 is configured with a triggering component (not shown) and the triggering signal provided by the triggering component may be transmitted through the signal transmitting circuit 112, the protection circuit 520 may receive the triggering signal through the signal receiving circuit 130 to generate a corresponding processing signal, such as a high logic level. Then, the processing signal with the high logic level may be transmitted to the switch 540, such that the switch 540 is turned on accordingly, so as to output the electrical stimulation signal generated by the signal processing circuit. When the external controller 110 is not configured with the triggering component, the protection circuit 520 may not receive the triggering signal to generate a corresponding processing signal, such as a low logic level. Then, the processing signal with the low logic level may be transmitted to the switch 540, such that the switch 540 is not turned on, and the protection circuit 520 does not output the electrical stimulation signal generated by the signal processing circuit 150. Therefore, the situation that the electrical stimulation device 510senses or receives the frequency signal generated by the other devices or emission sources to generate the error operation (for example, directly perform the electrical stimulation operation) may be effectively avoided, so as to increase the safety of the electrical stimulation device 510 and decrease the trouble of the user.

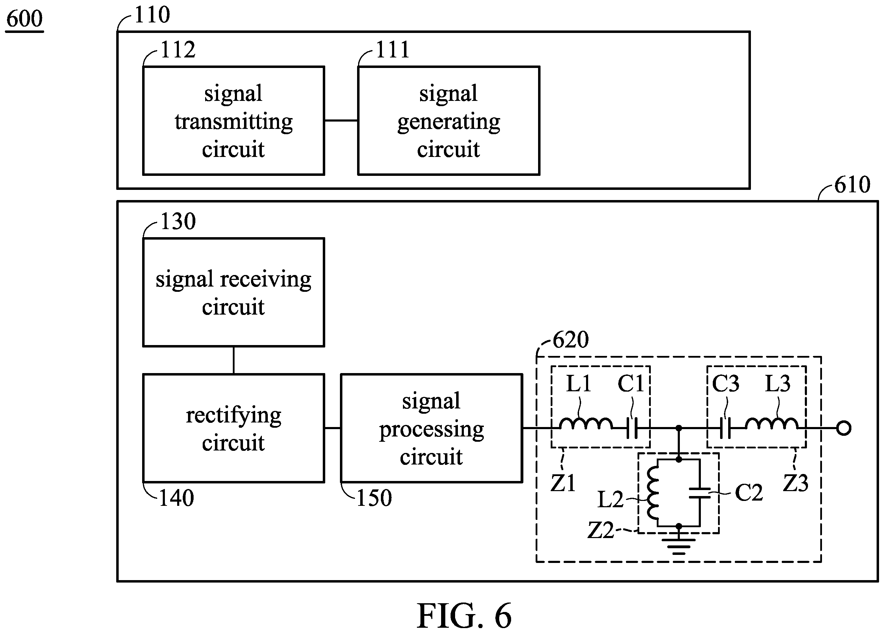

FIG. 6 is a schematic view of an electrical stimulation system according to another embodiment of the present disclosure. Please refer to FIG. 6. The electrical stimulation system 600 includes an external controller 110 and an electrical stimulation device 610. In the embodiment, the external controller 110 in FIG. 6 is the same as or similar to external controller 110 in FIG. 1. Accordingly, the external controller 110 in FIG. 6 may refer to the description of the embodiment of FIG. 1, and the description thereof is not repeated herein.

The electrical stimulation device 610 includes a signal receiving circuit 130, a rectifying circuit 140, a signal processing circuit 150 and a signal converting circuit 620. In the embodiment, the signal receiving circuit 130, the rectifying circuit 140 and the signal processing circuit 150 in FIG. 6 are the same as or similar to the signal receiving circuit 130, the rectifying circuit 140 and the signal processing circuit 150 in FIG. 1. Accordingly, the signal receiving circuit 130, the rectifying circuit 140 and the signal processing circuit 150 in FIG. 6 may refer to the embodiment of FIG. 1, and the description thereof is not repeated herein.

In the embodiment, the electrical stimulation signal generated by the signal processing circuit 150 is the square wave signal. The signal converting circuit 620 is coupled to the signal processing circuit 150. The signal converting circuit 620 is configured to receive the electrical stimulation signal (i.e., the square wave signal), and converts the electrical stimulation signal to generate a sine wave electrical stimulation signal. That is, sine wave electrical stimulation signal may be an electrical stimulation signal with a sine wave.

Furthermore, the signal converting circuit 620 is, for example, a passive filter. For example, the signal converting circuit 620 may include an impedance unit Z1, an impedance unit Z2 and an impedance unit Z3. The impedance unit Z1 includes a first terminal and a second terminal. The first terminal of the impedance unit Z1 is coupled to the signal processing circuit 150 and receives the electrical stimulation signal. The impedance unit Z2 includes a first terminal and a second terminal. The first terminal of the impedance unit Z2 is coupled to the second terminal of the impedance unit Z1. The second terminal of the impedance unit Z2 is coupled to a ground terminal. The impedance unit Z3 includes a first terminal and a second terminal. The first terminal of the impedance unit Z3 is coupled to the second terminal of the impedance unit Z1. The second terminal of the impedance unit Z3 generates the sine wave electrical stimulation signal.

Furthermore, the impedance unit Z1 may include an inductor L1 and a capacitor C1. The inductor L1 includes a first terminal and a second terminal. The first terminal of the inductor L1 serves as the first terminal of the impedance unit Z1, is coupled to the signal processing circuit 150 and receives the electrical stimulation signal. The capacitor C1 includes a first terminal and a second terminal. The first terminal of the capacitor C1 is coupled to the second terminal of the inductor L1. The second terminal of the capacitor C1 serves as the second terminal of the impedance unit Z1.

The impedance unit Z2 may include an inductor L2 and a capacitor C2. The inductor L2 includes a first terminal and a second terminal. The first terminal of the inductor L2 serves as the first terminal of the impedance unit Z2, and is coupled to the second terminal of the capacitor C1. The second terminal of the inductor L2 serves as the second terminal of the impedance unit Z2, and is coupled to the ground. The capacitor C2 includes a first terminal and a second terminal. The first terminal of the capacitor C2 is coupled to the first terminal of the inductor L2. The second terminal of the capacitor C2 is coupled to the second terminal of the inductor L2. The impedance unit Z3 may include a capacitor C3 and an inductor L3. The capacitor C3 includes a first terminal and a second terminal. The first terminal of the capacitor C3 serves as the first terminal of the impedance unit Z3, and is coupled to the second terminal of the capacitor C1. The inductor L3 includes a first terminal and a second terminal. The first terminal of the inductor L3 is coupled to the second terminal of the capacitor C3. The second terminal of the inductor L3 serves as the second terminal of the impedance unit Z3, and generates the sine wave electrical stimulation signal.

The electrical stimulation device 610 of the embodiment converts the electrical stimulation signal with the square wave signal generated by the signal processing circuit 150 into the sine wave electrical stimulation signal through the signal converting circuit 620, so as to increase the electrical stimulation effect of the electrical stimulation device 610 on the target area to be stimulated.

In some embodiments, the electrical stimulation device 610 may further include a protection circuit 160 as shown in FIG. 1. The protection circuit 160 may be coupled to the signal converting circuit 620 (such as the second terminal of the impedance unit Z3), and the protection circuit 160 is configured to determine whether to output the sine wave electrical stimulation signal generated by the signal converting circuit 620. In addition, the related operation and description of the protection circuit 160 may refer to the embodiment of FIG. 1, and the description thereof is not repeated herein. Therefore, the situation that the electrical stimulation device 610senses or receives the frequency signal generated by the other devices or emission sources to generate the error operation (for example, directly perform the electrical stimulation operation) may be effectively avoided, so as to increase the safety of the electrical stimulation device 610 and decrease the trouble of the user.

In some embodiments, the electrical stimulation device 610 may further include a protection circuit 520 as shown in FIG. 5. The protection circuit 520 may be coupled to the signal converting circuit 620 (such as the second terminal of the impedance unit Z3), and the protection circuit 520 is configured to determine whether to output the sine wave electrical stimulation signal generated by the signal converting circuit 620. In addition, the related operation and description of the protection circuit 520 may refer to the embodiment of the FIG. 5, and the description thereof is not repeated herein. Therefore, the situation that the electrical stimulation device 610senses or receives the frequency signal generated by the other devices or emission sources to generate the error operation (for example, directly perform the electrical stimulation operation) may be effectively avoided, so as to increase the safety of the electrical stimulation device 610 and decrease the trouble of the user.

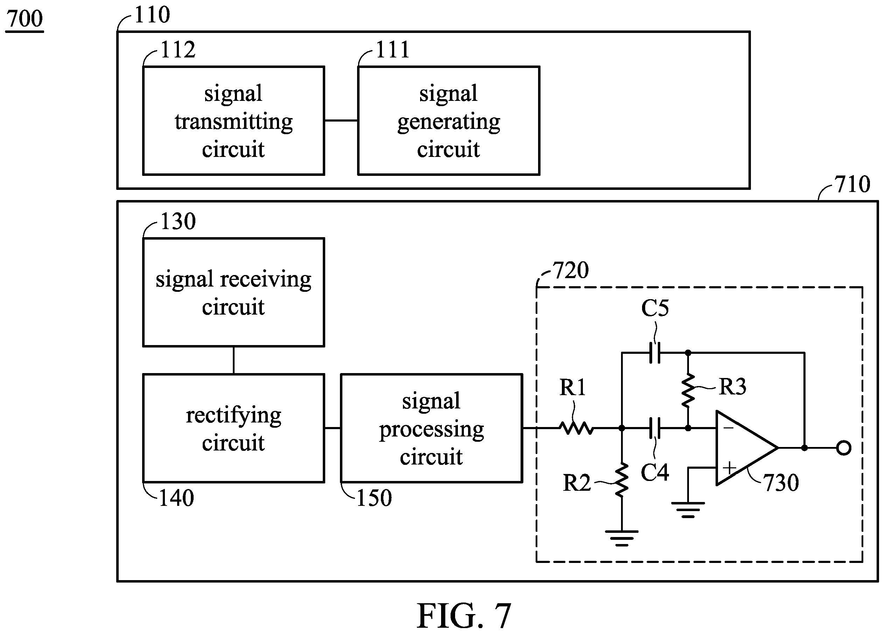

FIG. 7 is a schematic view of an electrical stimulation system according to another embodiment of the present disclosure. Please refer to FIG. 7. The electrical stimulation system 700 includes an external controller 110 and an electrical stimulation circuit 710. In the embodiment, the external controller 110 in FIG. 7 is the same as or similar to external controller 110 in FIG. 1. Accordingly, the external controller 110 in FIG. 7 may refer to the description of the embodiment of FIG. 1, and the description thereof is not repeated herein.

The electrical stimulation device 710 includes a signal receiving circuit 130, a rectifying circuit 140, a signal processing circuit 150 and a signal converting circuit 720. In the embodiment, the signal receiving circuit 130, the rectifying circuit 140 and the signal processing circuit 150 in FIG. 7 are the same as or similar to the signal receiving circuit 130, the rectifying circuit 140 and the signal processing circuit 150 in FIG. 1. Accordingly, the signal receiving circuit 130, the rectifying circuit 140 and the signal processing circuit 150 in FIG. 7 may refer to the embodiment of FIG. 1, and the description thereof is not repeated herein.

In the embodiment, the electrical stimulation signal generated by the signal processing circuit 150 is the square wave signal. The signal converting circuit 720 may be an active filter. For example, the signal converting circuit 720 may include a resistor R1, a resistor R2, a capacitor C4, a capacitor C5, a resistor R3 and an amplifier 730.

The resistor R1 includes a first terminal and a second terminal. The first terminal of the resistor R1 is coupled to the signal processing circuit 150, and receives the electrical stimulation signal. The resistor R2 includes a first terminal and a second terminal. The first terminal of the resistor R2 is coupled to the second terminal of the resistor R1. The second terminal of the resistor R2 is coupled to the ground terminal.

The capacitor C4 includes a first terminal and a second terminal. The first terminal of the capacitor C4 is coupled to the second terminal of the resistor R1. The capacitor C5 includes a first terminal and a second terminal. The first terminal of the capacitor C5 is coupled to the first terminal of the capacitor C4. The resistor R3 includes a first terminal and a second terminal. The first terminal of the resistor R3 is coupled to the second terminal of the capacitor C4. The second terminal of the resistor R3 is coupled to the second terminal of the capacitor C5.

The amplifier 730 includes a first input terminal, a second input terminal and an output terminal. The first input terminal (such as a positive input terminal) of the amplifier 730 is coupled to the ground terminal. The second input terminal (such as a negative input terminal of the amplifier 730 is coupled to the second terminal of the resistor R3. The output terminal of the amplifier 730 is coupled to the first terminal of the resistor R3, and outputs the sine wave electrical stimulation signal.

The electrical stimulation device 710 of the embodiment converts the electrical stimulation signal with the square wave signal generated by the signal processing circuit 150 into the sine wave electrical stimulation signal through the signal converting circuit 720, so as to increase the electrical stimulation effect of the electrical stimulation device 710 on the target area to be stimulated.

In some embodiments, the electrical stimulation device 710 may further include a protection circuit 160 as shown in FIG. 1. The protection circuit 160 may be coupled to the signal converting circuit 720 (such as the output terminal of the amplifier 730), and the protection circuit 160 is configured to determine whether to output the sine wave electrical stimulation signal generated by the signal converting circuit 720. In addition, the related operation and description of the protection circuit 160 may refer to the embodiment of FIG. 1, and the description thereof is not repeated herein. Therefore, the situation that the electrical stimulation device 710senses or receives the frequency signal generated by the other devices or emission sources to generate the error operation (for example, directly perform the electrical stimulation operation) may be effectively avoided, so as to increase the safety of the electrical stimulation device 710 and decrease the trouble of the user.

In some embodiments, the electrical stimulation device 710 may further include a protection circuit 520 as shown in FIG. 5. The protection circuit 520 may be coupled to the signal converting circuit 720 (such as the second input terminal of the amplifier 730), and the protection circuit 520 is configured to determine whether to output the sine wave electrical stimulation signal generated by the signal converting circuit 720. In addition, the related operation and description of the protection circuit 520 may refer to the embodiment of the FIG. 5, and the description thereof is not repeated herein. Therefore, the situation that the electrical stimulation device 710senses or receives the frequency signal generated by the other devices or emission sources to generate the error operation (for example, directly perform the electrical stimulation operation) may be effectively avoided, so as to increase the safety of the electrical stimulation device 710 and decrease the trouble of the user.

In summary, according to the electrical stimulation device and the electrical stimulation system disclosed by the present disclosure, the signal receiving circuit receives and outputs the frequency signal, the rectifying circuit receives the frequency signal and rectifies the frequency signal to generate the rectifying signal, and the signal processing circuit receives the rectifying signal to generate the electrical stimulation signal. Therefore, the complexity of circuit design may be effectively decreased, the size of the electrical stimulation device is decreased, and the convenience of use is increased.

In addition, when the electrical stimulation signal generated by the signal processing circuit is the square wave, the electrical stimulation device of the embodiment of the present disclosure further includes the signal converting circuit, and the signal converting circuit converts electrical stimulation signal with the square wave signal into the sine wave electrical stimulation signal (i.e., the electrical stimulation signal with the sine wave signal). Therefore, the electrical stimulation effect of the electrical stimulation device on the target area to be stimulated may be effectively increased. Furthermore, the electrical stimulation device of the embodiment of the present disclosure further includes the protection circuit. The protection circuit may be coupled to the signal processing circuit, and is configured to determine whether to output the electrical stimulation signal generated by the signal processing circuit, or the protection circuit may be coupled to the signal converting circuit, and is configured to determine whether to output the sine wave electrical stimulation signal generated by the signal converting circuit. Therefore, the situation that the electrical stimulation device senses or receives the frequency signal generated by the other devices or emission sources to generate the error operation (for example, directly perform the electrical stimulation operation) may be effectively avoided, so as to increase the safety of the electrical stimulation device and decrease the trouble of the user.

While the disclosure has been described by way of example and in terms of the preferred embodiments, it should be understood that the disclosure is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. An electrical stimulation device (120), comprising:
a signal receiving circuit (130), configured to receive and output a frequency signal;
a rectifying circuit (140), configured to receive the frequency signal and rectify the frequency signal to generate a rectifying signal; and
a signal processing circuit (150), configured to receive the rectifying signal to generate an electrical stimulation signal.

2. The electrical stimulation device (120) as claimed in claim 1, further comprising at least one electrode (221, 222), wherein the at least one electrode (221, 222) is coupled to the signal processing circuit (150).

3. The electrical stimulation device (120) as claimed in claim 1 or 2, wherein the signal processing circuit (150) is an oscillator and/or comprises a counter or a pulse width modulation circuit.

4. The electrical stimulation device (120) as claimed in any one of the preceding claims, wherein the electrical stimulation device (120) does not have a battery.

5. The electrical stimulation device (120) as claimed in any one of the preceding claims, further comprising a signal converting circuit (620), configured to receive the electrical stimulation signal, and to convert the electrical stimulation signal to generate a sine wave electrical stimulation signal

6. The electrical stimulation device as claimed in claim 5, wherein the signal converting circuit (620) is a passive filter and/or comprises:
a first inductor (L1), comprising a first terminal and a second terminal, wherein the first terminal of the first inductor (L1) is coupled to the signal processing circuit (150) and configured to receive the electrical stimulation signal;
a first capacitor (C1), comprising a first terminal and a second terminal, wherein the first terminal of the first capacitor (C1) is coupled to the second terminal of the first inductor (L1);
a second inductor (L2), comprising a first terminal and a second terminal, wherein the first terminal of the second inductor (L2) is coupled to the second terminal of the first capacitor, and the second terminal of the second inductor (L2) is coupled to a ground terminal;
a second capacitor (C2), comprising a first terminal and a second terminal, wherein the first terminal of the second capacitor (C2) is coupled to the first terminal of the second inductor (L2), and the second terminal of the second capacitor (C2) is coupled to the second terminal of the second inductor (L2);
a third capacitor (C3), comprising a first terminal and a second terminal, wherein the first terminal of the third capacitor (C3) is coupled to the second terminal of the first capacitor (C1); and
a third inductor (L3), comprising a first terminal and a second terminal, wherein the first terminal of the third inductor (L3) is coupled to the second terminal of the third capacitor (C3), and the second terminal of the third inductor (L3) is configured to generate the sine wave electrical stimulation signal.

7. The electrical stimulation device as claimed in claim 5, wherein the signal converting circuit (720) is an active filter and/or comprises:
a first resistor (R1), comprising a first terminal and a second terminal, wherein the first terminal of the first resistor (R1) is coupled to the signal processing circuit (150) and configured to receive the electrical stimulation signal;
a second resistor (R2), comprising a first terminal and a second terminal, wherein the first terminal of the second resistor (R2) is coupled to the second terminal of the first resistor, and the second terminal of the second resistor (R2) is coupled to a ground;
a first capacitor (C1), comprising a first terminal and a second terminal, wherein the first terminal of the first capacitor (C1) is coupled to the second terminal of the first resistor (R1);
a second capacitor, comprising a first terminal and a second terminal, wherein the first terminal of the second capacitor (C2) is coupled to the first terminal of the first capacitor (C1);
a third resistor (R3), comprising a first terminal and a second terminal, wherein the first terminal of the third resistor (R3) is coupled to the second terminal of the first resistor, and the second terminal of the third resistor (R3) is coupled to the second terminal of the second capacitor (C2); and
an amplifier (730), comprising a first input terminal, a second input terminal and an output terminal, wherein the first input terminal of the amplifier (730) is coupled to the ground terminal, the second input terminal of the amplifier (730) is coupled to the second terminal of the third resistor (R3), and the output terminal of the amplifier (730) is coupled to the first terminal of the third resistor (R3) and configured to output the sine wave electrical stimulation signal.

8. The electrical stimulation device as claimed in any one of the preceding claims, further comprising a protection circuit (160, 520), coupled to the signal processing circuit (150) and/or the protection circuit (160, 520) is configured to determine whether to output the electrical stimulation signal; and/or the protection circuit (160, 520) is a Hall switch or a magnetic reed switch.

9. The electrical stimulation device as claimed in claim 8, wherein the protection circuit (520) comprises a processing circuit (530), coupled to the signal receiving circuit (150), and configured to receive a triggering signal to generate a processing signal; and a switch (540), coupled to the signal processing circuit (150) and the processing circuit (530), and configured to receive the processing signal and determine whether to output the electrical simulation signal according to the processing signal.

10. The electrical stimulation device as claimed in claim 9, wherein the processing circuit(530) is a near field communication circuit, a radio frequency identification circuit, or an authenticator and security integrated circuit.

11. The electrical stimulation device as claimed in any one of the preceding claims, further comprising at least one electrode (221, 222), wherein the at least one electrode (221, 222) is coupled to the protection circuit (160, 520).

12. The electrical stimulation device as claimed in any one of the preceding claims, wherein the signal receiving circuit (130) comprises a coil, an antenna, an ultrasonic circuit, or a combination thereof.

13. The electrical stimulation device as claimed in any one of the preceding claims, wherein a frequency range of the frequency signal comprises a range of 1 MHz to 1.2 MHz, a range of 9 KHz to 200 KHz, or a range of 6 MHz to 45 MHz.

14. The electrical stimulation device (120) as claimed in any one of the preceding claims, wherein a frequency of the frequency signal is 13.56 MHz; and/or a frequency range of the electrical simulation signal is 100 KHz to 2000 KHz; and/or a frequency range of the electrical simulation signal is 480 KHz to 520 KHz and/or a voltage range of the electrical stimulation signal is between -25 V and 25 V and/or a current range of the electrical simulation signal is between 0 and 60 mA.

15. An electrical stimulation system, comprising:
an external controller (110), comprising:
a signal generating circuit (111), configured to generate a frequency signal; and
a signal transmitting circuit (112), configured to receive and transmit the frequency signal; and
an electrical stimulation device (120) as claimed in any one of the preceding claims.
